# EUROPEAN PATENT APPLICATION

(11) **EP 4 483 900 A1**
(43) Date of publication of application: **01.01.2025**
(21) Application number: 23759873.5
(22) Date of filing: 17.02.2023
(51) Int. Cl.: A61K 45/00, A61K 31/045, A61P 35/00, C12Q 1/06, G01N 33/15, G01N 33/50

(54) **ANTICANCER DRUG AND ANTICANCER DRUG SCREENING METHOD**

(30) Priority: 22.02.2022 JP 2022025433
(71) Applicant: Institute of Rheological Function of Food Co., Ltd., Kasuya-gun, Fukuoka 811-2501 (JP); Kyushu University, National University Corporation, Nishi-ku Fukuoka-shi Fukuoka 819-0395 (JP)
(72) Inventor: FUJINO Takehiko, Fukuoka-shi, Fukuoka 813-0043 (JP); MAWATARI Shiro, Fukuoka-shi, Fukuoka 813-0016 (JP); HONSHO Masanori, Fukuoka-shi, Fukuoka 819-0395 (JP); OKAUCHI Tatsuo, Kitakyushu-shi, Fukuoka 804-8550 (JP)
(74) Representative: V.O.
(86) International application number: PCT/JP2023/005654
(87) International publication number: WO 2023/162872

(57) **Abstract**

The anticancer agent of the disclosure contains an ATP8B2 inhibitor.

## Description

### Technical Field

The present disclosure relates to an anticancer agent and an anticancer agent screening method.

### Background Art

Plasmalogens are phospholipids with an antioxidant effect and are a subclass of glycerophospholipids. Plasmalogens are present in all mammalian tissues and account for about 18% of the phospholipids in the human body.

Since most plasmalogens are bound to polyunsaturated fatty acids such as docosahexaenoic acid and arachidonic acid, they are involved in storing polyunsaturated fatty acids, releasing secondary messengers of intercellular signals, such as prostaglandins and leukotrienes, produced from polyunsaturated fatty acids, and the like. Further, plasmalogens are involved in cell fusion, ion transport, and the like. Plasmalogens also serve a cellular antioxidant function, as they have a vinyl ether bond (alkenyl bond) that is particularly sensitive to oxidative stress. Furthermore, plasmalogens are known to have effects such as promoting neurogenesis, suppressing neuroinflammation caused by lipopolysaccharide (LPS), and suppressing accumulation of amyloid β (Aβ) protein in the brain.

Plasmalogens are known to be decreased in neurological diseases such as dementia, Parkinson's disease, depression, and schizophrenia, as well as in diabetes, metabolic syndrome, and ischemic heart disease. For example, Non Patent Literature 1 reported that ethanolamine-type plasmalogens are significantly decreased in the frontal lobe and hippocampus in Alzheimer's disease brains. Furthermore, Non Patent Literature 2 reports that plasmalogens are decreased in the serum of Alzheimer's disease patients. Non Patent Literature 3 reports that choline-type plasmalogens are decreased in a patient group with ischemic heart disease compared to a normal control group.

### Citation List

### Non Patent Literature

Non Patent Literature 1: Z. Guan et al., Decrease and Structural Modifications of Phosphatidylethanolamine Plasmalogen in the Brain with Alzheimer Disease, J Neuropathol Exp Neurol, 58(7), 740-747, 1999
Non Patent Literature 2: D. B. Goodenowe et al., Peripheral ethanolamine plasmalogen deficiency: a logical causative factor in Alzheimer's disease and dementia, J Lipid Res, 48, 2485-2498, 2007
Non Patent Literature 3: Takeo Sanada et al., Serum Plasmalogens in Ischemic Heart Disease, the Journal of Japan Atherosclerosis Society 11, 535-539, 1983

### Summary of Invention

### Technical Problem

Plasmalogen having ethanolamine in its polar head is known to be present abundantly in the inner leaflet of the cell membrane. It is inferred that the ubiquitous distribution of plasmalogen in the cell membrane is essential for cell function. However, the molecular mechanism for maintaining this ubiquitous distribution of plasmalogens in the cell membrane is unknown. It is believed that cell behavior can be controlled if the factors involved in this ubiquitous distribution of plasmalogen can be identified.

The present disclosure has been made given the above-described circumstances. An objective of the disclosure is to provide a novel anticancer agent and a novel anticancer agent screening method targeting a factor responsible for the ubiquitous distribution of plasmalogen in the cell membrane.

### Solution to Problem

The present inventors identified ATP8B2 belonging to P4-ATPase as a factor responsible for the ubiquitous distribution of plasmalogen in the cell membrane. The present inventors further found that the impaired ubiquitous distribution due to inhibiting ATP8B2 suppresses AKT activation, which is essential for cell survival. This has led to the completion of the disclosure.

An anticancer agent according to a first aspect of the disclosure contains an ATP8B2 inhibitor.

The ATP8B2 inhibitor may be 9-(1-octylcyclopropyl)nonan-1-ol, (S)-3-((9-(1-octylcyclopropyl)nonyl)oxy)propane-1,2-diol, or a pharmacologically acceptable salt thereof.

An anticancer agent screening method according to a second aspect of the disclosure comprises
an exposure step of exposing a cancer cell to a test substance,
a quantification step of quantifying a plasmalogen present in an outer leaflet of a cell membrane of the cancer cell, and
a step of comparing an amount of the plasmalogen quantified in the quantification step with an amount of a control.

### Advantageous Effects of Invention

According to the disclosure, a novel anticancer agent that targets a factor responsible for the ubiquitous distribution of plasmalogens in the cell membrane can be obtained.

### Brief Description of Drawings

FIG. 1 illustrates the impaired ubiquitous distribution of plasmalogens in the cell membrane due to suppressing ATP8B2 expression according to Example 1, FIG. 1A illustrates the results of thin-layer chromatography (TLC), FIG. 1B illustrates the relative amount of plasmalogens modified with a membrane-impermeable compound in cells transfected with siRNA, FIG. 1C illustrates the relative amount of TNBS-modified phosphatidylethanolamine (PE) in cells transfected with siRNA;
FIG. 2 illustrates the effects of suppressing ATP8B2 expression on AKT activation according to Example 2, FIG. 2A illustrates the results of Western blotting, FIG. 2B illustrates the relative value of pAKT/AKT in cells transfected with siRNA;
FIG. 3 illustrates the total protein amount in cells according to Example 3;
FIG. 4 illustrates the appearance of a medium in which cells according to Example 3 were cultured;
FIG. 5 illustrates the effects of Compounds 1 to 3 according to Example 4 on AKT activation, FIG. 5A illustrates the results of Western blotting, FIG. 5B illustrates the relative value of pAKT(T308)/AKT, FIG. 5C illustrates the relative value of pAKT(S473)/AKT; and
FIG. 6 illustrates the effects of Compound 4 according to Example 4 on AKT activation, FIG. 6A illustrates the results of Western blotting, FIG. 6B illustrates the relative value of pAKT(T308)/AKT, FIG. 6C illustrates the relative value of pAKT(S473)/AKT.

### Description of Embodiments

The embodiments according to the present disclosure are described with reference to the drawings. The disclosure is not limited to the following embodiments and drawings. In the following embodiments, expressions such as "having," "including," and "containing" also include the meanings of "consisting of" and "comprising."

### Example 1

An anticancer agent according to the present embodiment contains an ATP8B2 inhibitor. The ATP8B2 inhibitor is not particularly limited as long as it inhibits ATP8B2 directly or indirectly. Examples of the ATP8B2 inhibitor include compounds, antibodies, proteins, peptides, DNA aptamers, and RNA aptamers that bind to ATP8B2 and suppress the activity of ATP8B2. The ATP8B2 inhibitor may inhibit ATP8B2 through suppressing the expression of the ATP8B2 gene. In this case, the ATP8B2 inhibitor is siRNA, antisense nucleic acid, or the like.

The ATP8B2 inhibitor can be identified by a binding assay targeting ATP8B2 or the like. ATP8B2 has flippase activity that transports phospholipids constituting a lipid bilayer membrane to the opposite side of the membrane. Therefore, a test substance that increases plasmalogens present in the outer leaflet of the cell membrane may be used as an ATP8B2 inhibitor. Whether plasmalogens present in the outer leaflet of the cell membrane have increased can be determined based on the amount of plasmalogens present in the outer leaflet of the cell membrane of a cell that has not been exposed to the test substance.

The ATP8B2 inhibitor is preferably a compound. A preferred compound as the ATP8B2 inhibitor is an aliphatic alcohol, which is specifically 9-(1-octylcyclopropyl)nonan-1-ol, (S)-3-((9-(1-octylcyclopropyl)nonyl)oxy)propane-1,2-diol, a pharmacologically acceptable salt thereof, or the like. The salt is not particularly limited as long as it is a pharmacologically acceptable salt and may be either an acidic salt or a basic salt. Examples of the salt include: alkali metal salts such as lithium, sodium, and potassium salts; alkaline earth metal salts such as magnesium and calcium salts; inorganic acid salts such as hydrochlorides, hydrobromides, sulfates, nitrates, oxalates, and phosphates; and organic acid salts such as acetate, propionate, hexanoate, cyclopentane propionate, glycolate, pyruvate, lactate, malonate, succinate, malate, fumarate, tartrate, citrate, benzoate, o-(4-hydroxybenzoyl)benzoate, cinnamate, mandelate, methanesulfonate, ethanesulfonate, 1,2-ethanedisulfonate, 2-hydroxyethanesulfonate, benzene sulfonate, p-chlorobenzene sulfonate, 2-naphthalene sulfonate, p-toluene sulfonate, camphor sulfonate, glucoheptanoate, 3-phenylpropionate, trimethyl acetate, tertiary butyl acetate, lauryl sulfate, gluconate, glutamate, hydroxynaphthoate, salicylate, stearate, trifluoroacetic acid (TFA) salt, maleate, and muconate.

A cancer for which the anticancer agent according to the present embodiment is effective is a cancer in which the PI3 (Phosphoinositide 3-kinase) kinase/Akt pathway is activated and Akt protein phosphorylation is increased, that is, the intracellular concentration of phosphorylated Akt protein is increased compared to that of the corresponding non-cancerous cells.

Examples of cancers targeted by the anticancer agent include cervical cancer, lymphoma, pancreatic cancer, brain tumor, pituitary tumor, esophageal cancer, leukemia, and breast cancer.

The anticancer agent according to the present embodiment is produced by a known method and contains an ATP8B2 inhibitor as an active ingredient. The anticancer agent contains, for example, from 0.1% to 99% by weight, from 1% to 50% by weight, preferably from 1% to 20% by weight of an ATP8B2 inhibitor as an active ingredient.

The dosage form of the anticancer agent according to the present embodiment is preferably an injection or an oral dosage form. In addition, the dosage form of the anticancer agent may be a rectal suppository, a vaginal suppository, a nasal absorption agent, a transdermal absorption agent, a pulmonary absorption agent, an oral absorption agent, or the like. The anticancer agent may be a combination drug compounded with a pharmacologically acceptable carrier. Pharmaceutically acceptable carriers are various organic or inorganic carrier materials. A pharmacologically acceptable carrier is compounded as, for example, an excipient, lubricant, binder, or disintegrant in a solid preparation or a solvent, solubilizing agent, suspending agent, tonicity agent, buffering agent, or soothing agent in a liquid preparation into the anticancer agent. In addition, additives such as a preservative, an antioxidant, a colorant, and a sweetener can also be used as necessary.

Examples of the excipient include lactose, white sugar, D-mannitol, starch, crystalline cellulose, and light anhydrous silicic acid. Examples of the lubricant include magnesium stearate, calcium stearate, talc, and colloidal silica. Examples of the binder include crystalline cellulose, white sugar, D-mannitol, dextrin, hydroxypropylcellulose, hydroxypropylmethylcellulose, and polyvinylpyrrolidone. Examples of the disintegrant include starch, carboxymethyl cellulose, carboxymethyl cellulose calcium, croscarmellose sodium, and carboxymethyl starch sodium.

Examples of the solvent include water for injection, alcohol, propylene glycol, and macrogol. Examples of the solubilizing agent include polyethylene glycol, propylene glycol, D-mannitol, benzyl benzoate, ethanol, trisaminomethane, cholesterol, triethanolamine, sodium carbonate, and sodium citrate. The suspending agent is a surfactant, a hydrophilic polymer, or the like. Examples thereof include stearyltriethanolamine, sodium lauryl sulfate, lauryl aminopropionic acid, lecithin, benzalkonium chloride, benzethonium chloride, glyceryl monostearate, polyvinyl alcohol, polyvinylpyrrolidone, sodium carboxymethylcellulose, methylcellulose, hydroxymethylcellulose, hydroxyethylcellulose, and hydroxypropylcellulose.

Examples of the tonicity agent include sodium chloride, glycerin, and D-mannitol. Examples of the buffering agent include phosphate, acetate, carbonate, and citrate buffers. Examples of the soothing agent include benzyl alcohol. Examples of the preservative include paraoxybenzoic acid esters, chlorobutanol, benzyl alcohol, phenethyl alcohol, dehydroacetic acid, and sorbic acid. Examples of the antioxidant include sulfite and ascorbic acid.

Subjects to which the anticancer agent according to the present embodiment is administered are humans and non-human animals. The animals are preferably mammals. More specific examples thereof include dogs, cats, cows, pigs, horses, sheep, and deer.

The dosage of the anticancer agent according to the present embodiment is appropriately determined depending on the patient's sex, age, weight, symptoms, and the like. The anticancer agent is administered in such a way that the ATP8B2 inhibitor is administered in a therapeutically effective amount. The effective amount is the amount of ATP8B2 inhibitor necessary to achieve the desired results, which is required to slow the advancement of, inhibit, prevent, reverse, or cure the condition being treated or managed. The dosage of the anticancer agent is typically from 0.01 to 1000 mg/kg, preferably from 0.1 to 200 mg/kg, more preferably from 0.2 to 20 mg/kg. It can be administered once or in one or more divided doses daily. The anticancer agent may be administered at various dosing frequencies, such as daily, every other day, once a week, every other week, and once a month. If necessary, the anticancer agent may be used in amounts outside the above-described ranges.

The method of administering the anticancer agent according to the present embodiment is not particularly limited, but the anticancer agent may be administered by injection, nasally, transdermally, pulmonaryly, orally, or the like. As the administration method, intravenous administration, intraarterial administration, or oral administration is particularly preferable.

The anticancer agent according to the present embodiment is a novel anticancer agent that targets a factor responsible for the ubiquitous distribution of plasmalogens in the cell membrane. As illustrated in the Examples below, the anticancer agent suppresses cancer cell proliferation by suppressing AKT phosphorylation through the impaired ubiquitous distribution of plasmalogens.

In other embodiments, the use of an ATP8B2 inhibitor for the production of an anticancer agent is provided. In another embodiment, a method of treating, ameliorating, or preventing a cancer is provided. The method comprises administering an ATP8B2 inhibitor to a subject. In other embodiments, an ATP8B2 inhibitor is also provided for use in the treatment of a cancer.

### Example 2

The anticancer agent screening method according to the present embodiment comprises an exposure step, a quantification step, and a comparison step. A cancer cell is exposed to a test substance in the exposure step. The cancer cell may be a primary cell collected from a cancer tissue or may be of a cancer-derived cell line. As the test substance, a compound included in a library of compounds, or the like can be used. In the exposure step, a test substance may be added to, for example, a medium containing a cancer cell.

In the quantification step, plasmalogens present in the outer leaflet of the cell membrane of the cancer cell are quantified. For example, by modifying the amino groups of plasmalogens present in the outer leaflet of the cell membrane with a membrane-impermeable compound, plasmalogens present in the outer leaflet of the cell membrane and plasmalogens present outside the outer leaflet can be detected separately. Examples of the membrane-impermeable compound include 2,4,6-trinitrobenzenesulfonic acid (TNBS). Plasmalogens can be detected by a known method. To quantify plasmalogens present in the outer leaflet, for example, first, all lipids are extracted from a precipitate obtained by centrifuging a cell lysate in which cancer cells are solubilized using the Bligh and Dyer method or a similar method. Subsequently, plasmalogens modified with a membrane-impermeable compound are quantified by various chromatographies such as TLC and liquid chromatography.

In the comparison step, the amount of plasmalogens quantified in the quantification step is compared with a control. The control may be the amount of plasmalogens present in the outer leaflet of the cell membrane of a cancer cell that has not been exposed to the test substance or the amount of plasmalogens present in the outer leaflet of the cell membrane of a cancer cell that has been exposed to a positive control substance exhibiting a growth-inhibitory effect on a cancer cell. By comparing with a positive control, it is possible to more reliably obtain a test substance that exhibits a growth-inhibiting effect on a cancer cell. In the comparison step, an appropriate value such as the average value ± standard deviation may be used as the control amount from the previously measured amount of plasmalogens present in the outer leaflet of the cell membrane of a cancer cell that has not been exposed to a test substance.

A test substance that significantly increases the amount of plasmalogens present in the outer leaflet of the cell membrane compared to the control is selected as a candidate for the anticancer agent. Here, the expression "significantly increase" means increasing with a statistically significant difference. To assess a statistical difference, a difference between the average amount of plasmalogens present in the outer leaflet of the cell membrane of a cancer cell that has been exposed to a test substance and the average amount of plasmalogens present in the outer leaflet of the cell membrane of a cancer cell that has not been exposed to a test substance can be tested by a statistical testing method such as a t-test.

According to the anticancer agent screening method according to the present embodiment, it is possible to obtain a novel anticancer agent that targets a factor responsible for the ubiquitous distribution of plasmalogens in the cell membrane.

### Examples

The disclosure is explained in more detail with reference to the following Examples; however, it is not limited by these Examples.

### Example 1: Examination of Suppression of ATP8B2 Expression

The effect of suppressing the expression of ATP8B2 on the localization of plasmalogens was examined. As siRNA that suppresses the expression of ATP8B2, Mission (trademark) siRNA (SASI_Hs01_00037709 manufactured by Merck KGaA, SEQ ID NO: 1) was introduced into HeLa cells and cultured for 54 hours. The medium composition included Dulbecco's Modified Eagle Medium (manufactured by Sigma-Aldrich), 10% fetal calf serum (FCS, manufactured by Sigma-Aldrich), and 100 units/mL penicillin and 100 µg/mL streptomycin (manufactured by NACALAI TESQUE, INC.).

Subsequently, plasmalogens (PlsEtn) present in the outer leaflet of the cell membrane were metabolically labeled with ¹⁴C-ethanolamine for 18 hours. Further, after washing with cold phosphate-buffered saline (PBS), the plasmalogens were equilibrated with a buffer containing 0.25 M sucrose, 10 mM Hepes-KOH (pH 8.5), and 1 µg/mL taxol (manufactured by Sigma-Aldrich). The amino groups of the plasmalogens were then treated with 10 mM TNBS, a membrane-impermeable compound, for 30 minutes on ice. Thereafter, unreacted TNBS was inactivated by treatment with Tris-HCl (pH 8.0) at a final concentration of 50 mM for 15 minutes. Thus, plasmalogens present on the cell surfaces were chemically modified.

Cells were collected, and cell lysates of equivalent amounts of protein from non-siRNA-transfected cells and siRNA-transfected cells were prepared using a cell lysate reagent (0.25M sucrose, 10 mM Hepes-KOH (pH 7.4), and 1 mM EDTA). Trichloroacetic acid was added to each cell lysate at a final concentration of 5%, which was left to stand for 30 minutes. A precipitate was obtained by centrifugation. After suspending the precipitate in PBS, all lipids were extracted by the Bligh and Dyer method and developed by TLC for 1 hour. The developing solvent for TLC was chloroform methanol: acetic acid = 65:25:10.

### (Results)

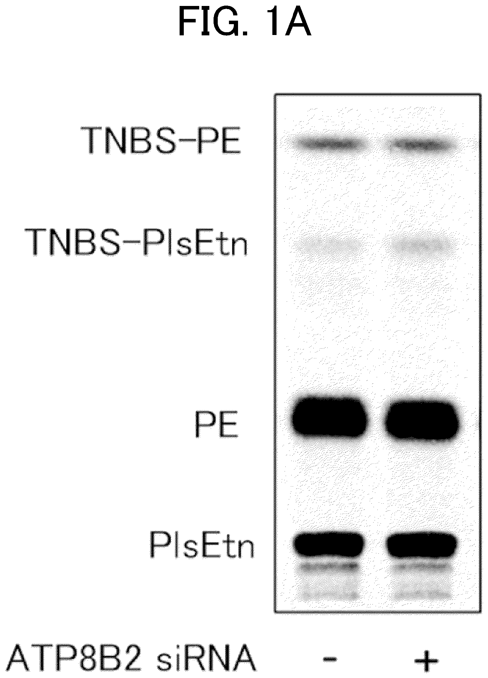

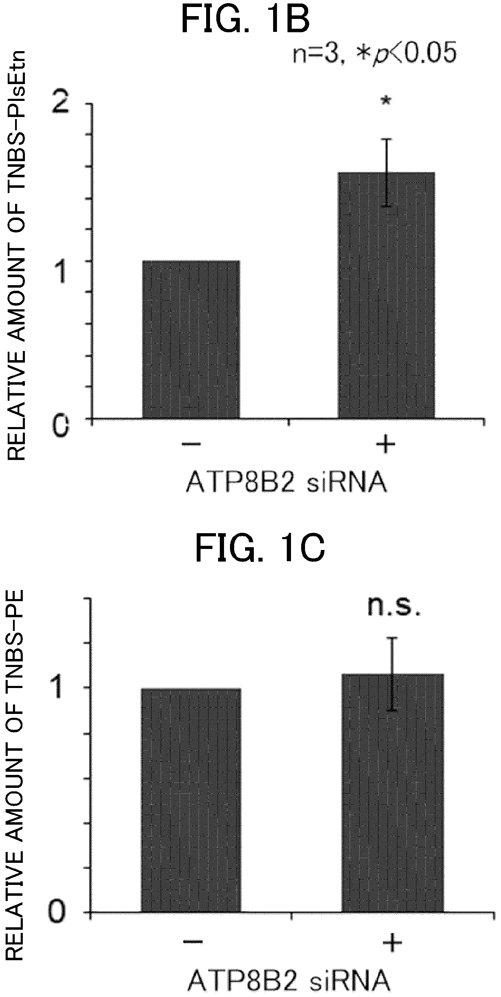

FIG. 1A illustrates the bands obtained by TLC. Suppression of ATP8B2 expression increased the TNBS-modified plasmalogen (TNBS-PlsEtn). FIG. 1B illustrates the relative amount of TNBS-PlsEtn in cells transfected with siRNA, as determined by quantifying the bands illustrated in FIG. 1A using Image J. The increase in TNBS-PlsEtn due to suppression of ATP8B2 expression was statistically significant. FIG. 1C illustrates the relative amount of PE modified with TNBS (TNBS-PE) in cells transfected with siRNA. There was no significant difference in the amount of TNBS-PE even when ATP8B2 expression was suppressed. The results showed that suppression of ATP8B2 expression increased extracellular exposure of PlsEtn.

### Example 2: Effects on AKT activation by Suppressing ATP8B2 Expression

Mission (trademark) siRNA (SASI_Hs01_00037709) was transfected into HeLa cells (1.5 × 10⁵ cells) and cultured for 72 hours. Cell lysates were prepared in the same manner as in Example 1. Phosphorylated AKT and AKT were each detected by Western blotting using SDS-PAGE with anti-AKT (T308) antibody (manufactured by Cell Signaling) and anti-AKT antibody (manufactured by Cell Signaling).

### (Results)

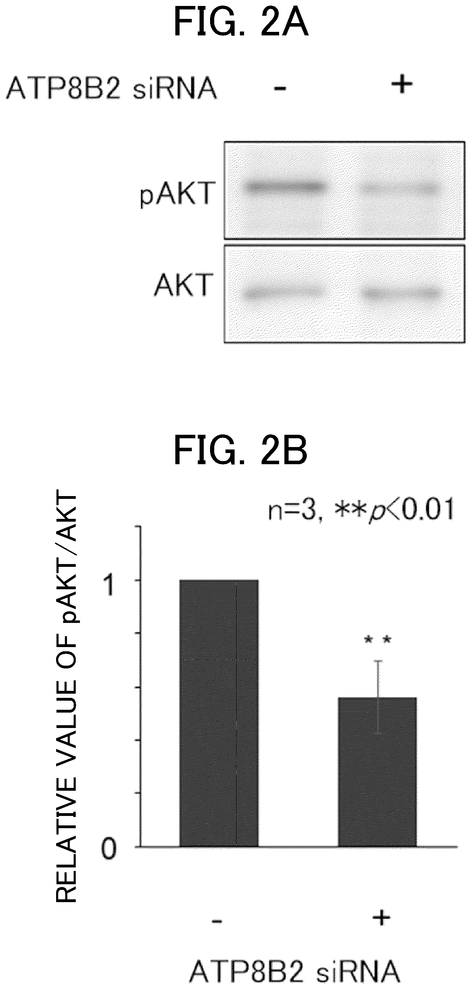

FIG. 2A illustrates pAKT and AKT bands obtained by Western blotting. FIG. 2B illustrates the relative value of pAKT/AKT in cells transfected with siRNA, as determined by quantifying the bands illustrated in FIG. 2A using Image J. Phosphorylation of AKT was suppressed by suppressing the expression of ATP8B2. The results showed that increased extracellular exposure of PlsEtn suppresses the phosphorylation of AKT.

### Example 3: Effects on Cell Proliferation due to Suppression of ATP8B2 Expression

siRNA against ATP8B2 was transfected into 1.5 × 10⁵ HeLa cells and cultured for 72 hours. The total amount of protein in the cells was quantified using a BCA Protein Assay Kit (manufactured by Thermo Fisher Scientific) using bovine serum albumin (BSA) as a standard. Since the medium becomes acidic as the cells grow, the color change (from red to yellow) of a phenol red pH indicator in the medium serves as an indicator of cell proliferation. A change in the color of the phenol red pH indicator in the medium was observed.

### (Results)

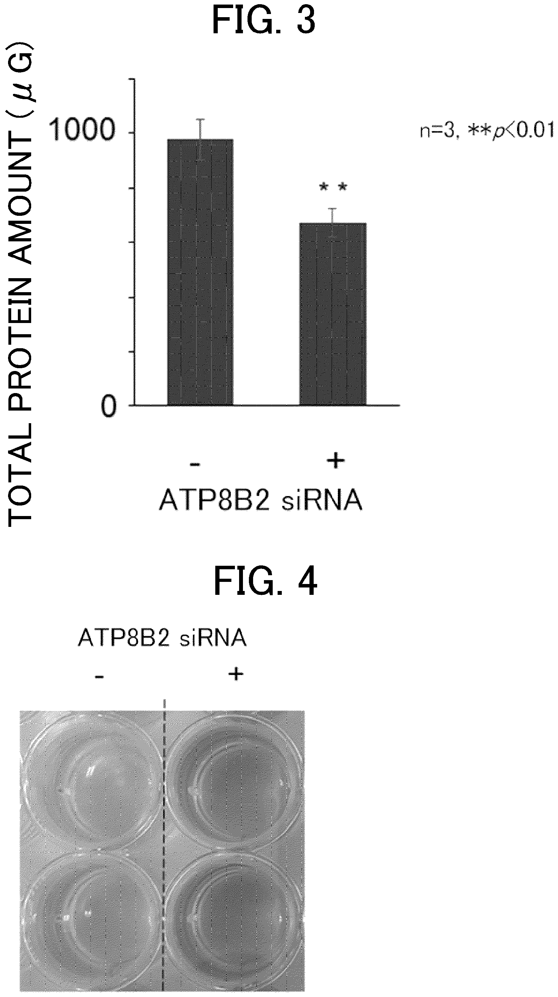

As illustrated in FIG. 3, the total protein amount was decreased, and cell proliferation was suppressed in the siRNA-transfected cells (+) compared to the non-siRNA-transfected cells (-). As illustrated in FIG. 4, the medium in the non-siRNA-transfected cells turned yellow, whereas the siRNA-transfected cells showed almost no change in color. Acidification of the medium was suppressed by suppressing the expression of ATP8B2. In other words, cell proliferation was suppressed by suppressing ATP8B2 expression.

### Example 4: Suppression of AKT Activation by Aliphatic Alcohol

### (Synthesis of Aliphatic Alcohol)

The plasmalogen biosynthesis pathway involves a step in which the acyl chain of acyl dihydroxyacetone phosphate is substituted with an aliphatic alcohol. Therefore, it is considered that by incorporating an aliphatic alcohol with a modifying group into cells, a plasmalogen having a modifying group at the sn-1 position is generated through the plasmalogen biosynthesis pathway. A plasmalogen having a modifying group at the sn-1 position has vinyl ether and is therefore recognized by ATP8B2 but is expected to inhibit the function of ATP8B2 due to the modifying group. Therefore, Compounds 1 to 3 were synthesized as three types of aliphatic alcohols having modifying groups at different sites as follows.

Compound 1 (5-(1-dodecylcyclopropyl)pentan-1-ol) was synthesized using ε-caprolactone as a starting material through the following reaction steps. Me, TBSCl, ^{t}BuOK, Et₂O, Ph, Et, THF, DMSO, TBS, and r.t. in the following reaction steps represent methyl group, tert-butyldimethylchlorosilane, potassium tert-butoxide, diethyl ether, phenyl group, ethyl group, tetrahydrofuran, dimethyl sulfoxide, tert-butyldimethylsilyl group, and room temperature, respectively.

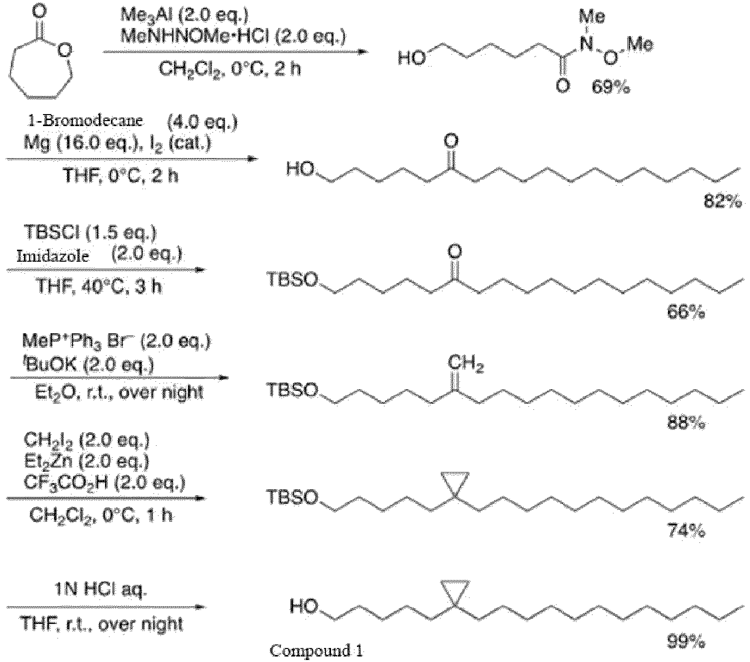

Compound 2 (4-(1-tridecylcyclopropyl)butan-1-ol) was synthesized using δ-valerolactone as a starting material through the following reaction steps.

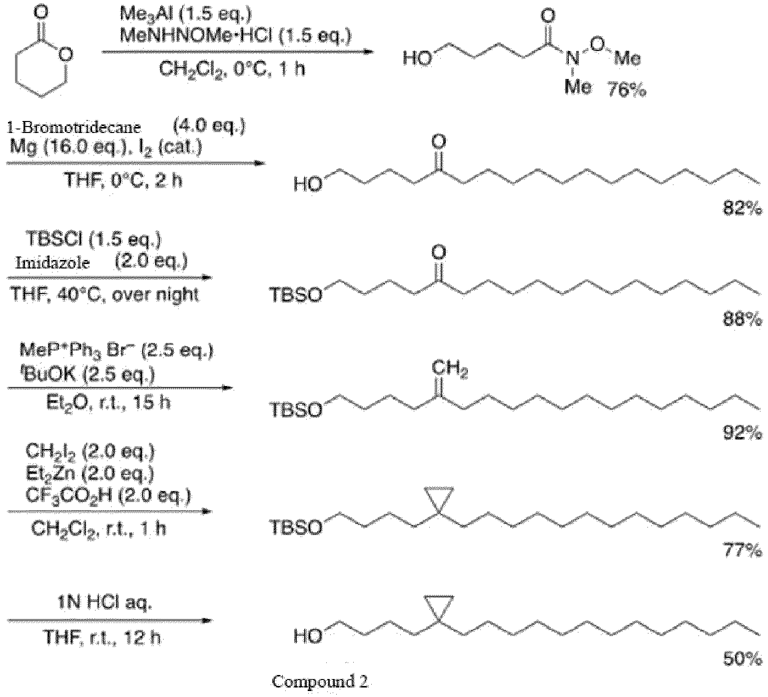

Compound 3 (9-(1-octylcyclopropyl)nonan-1-ol) was synthesized using 1,10-decanediol as a starting material through the following reaction steps.

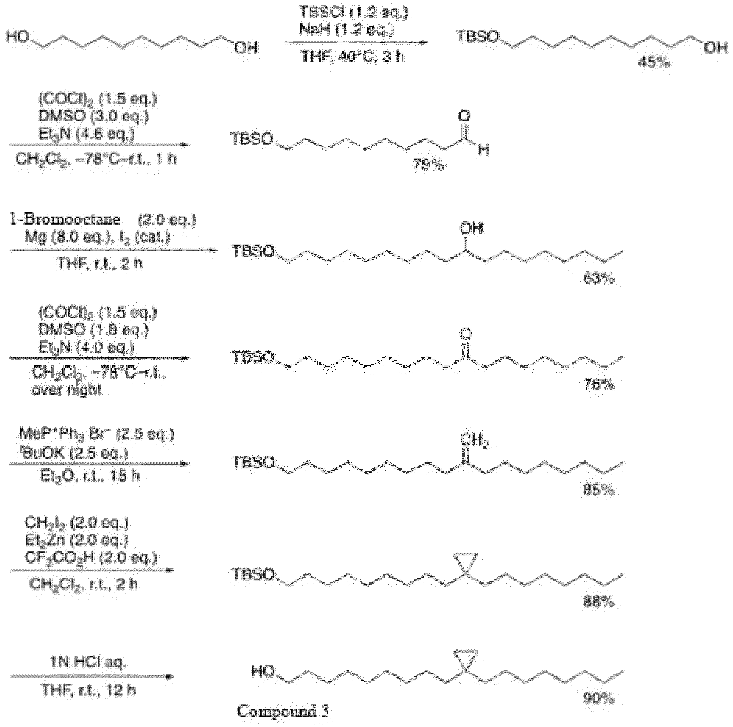

Compound 4 ((S)-3-((9-(1-octylcyclopropyl)nonyl)oxy)propane-1,2-diol) was synthesized using Compound 3 as a starting material through the following reaction steps. TsCl, TsO, and TsOH in the following reaction steps represent p-toluenesulfonyl chloride, p-toluenesulfonyloxy group, and p-toluenesulfonic acid, respectively.

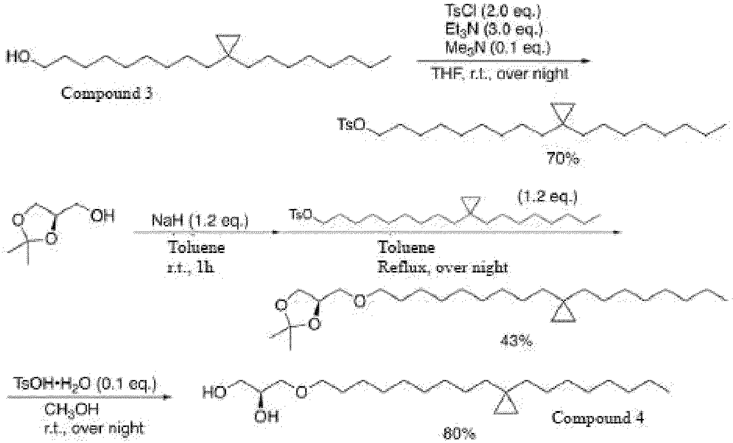

### (Examination of AKT Activation)

HeLa cells (5 × 10⁵ cells) were cultured for 48 hours in the presence of 20 µM Compound 1, Compound 2, Compound 3, or Compound 4. Compound 1, Compound 2, Compound 3, or Compound 4 was added at a concentration of 20 µM at the start of culture and 24 hours later. After the culture, cell lysates were prepared in the same manner as in Example 1. Phosphorylated AKT and AKT were detected by Western blotting using SDS-PAGE with an anti-AKT (T308) antibody (manufactured by Cell Signaling), an anti-AKT (S473) antibody (manufactured by Cell Signaling), and an anti-AKT antibody (manufactured by GeneTex).

### (Results)

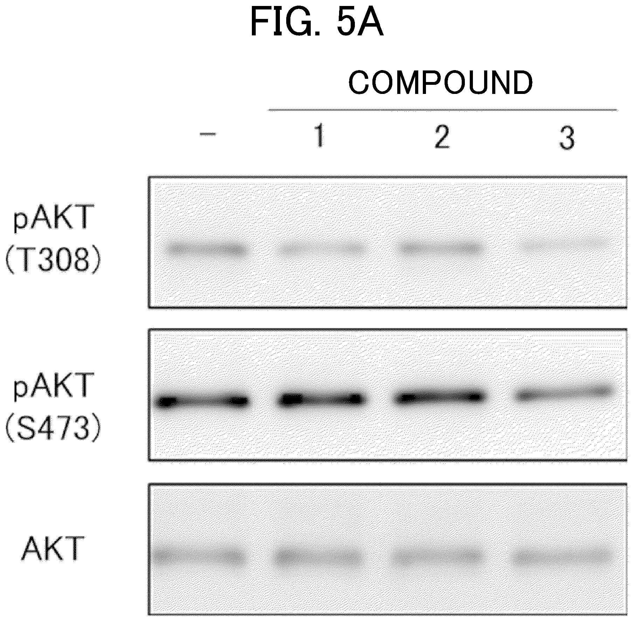

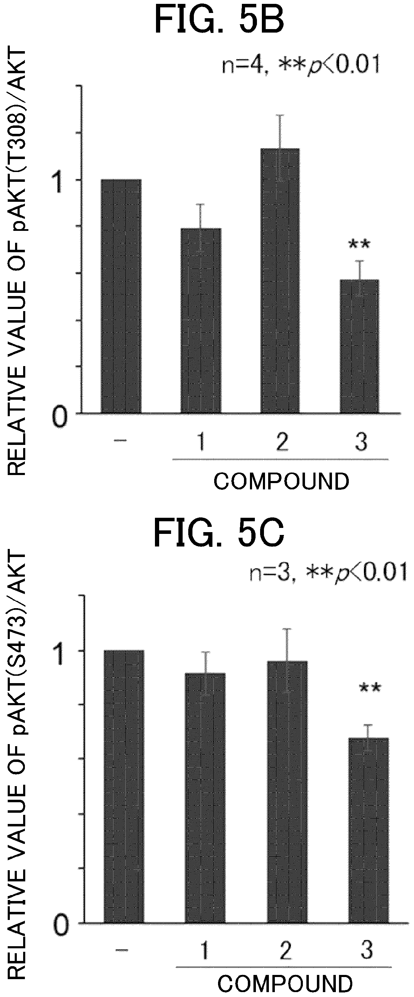

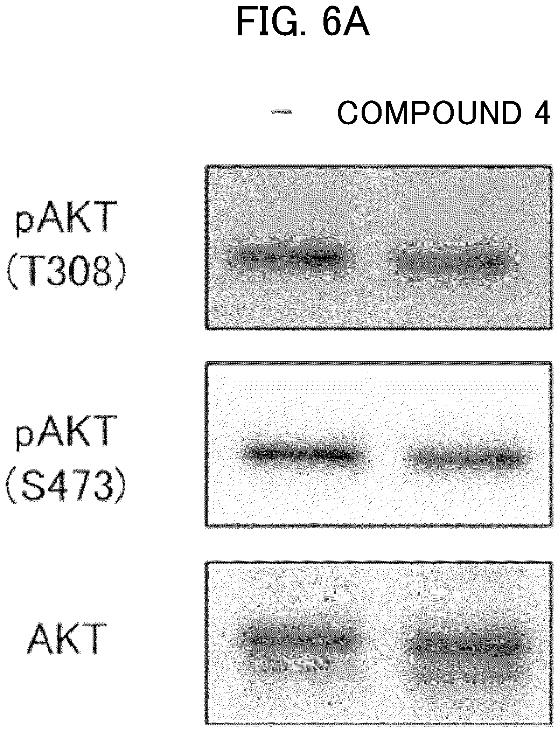

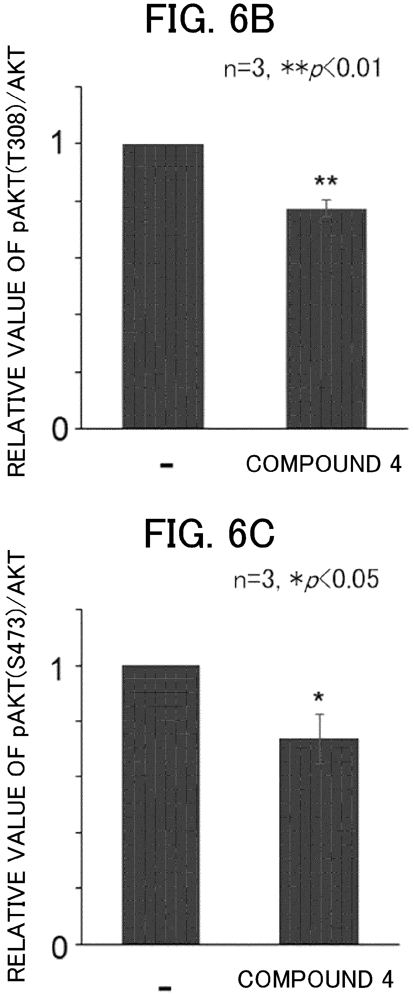

FIG. 5A illustrates pAKT and AKT bands obtained by Western blotting for Compound 1, Compound 2, and Compound 3. FIGS. 5B and 5C illustrate the relative values of pAKT(T308)/AKT and pAKT(S473)/AKT, respectively, which were quantified using Image J for the bands illustrated in FIG. 5A. FIG. 6A similarly illustrates bands of pAKT and AKT for Compound 4. FIGS. 6B and 6C illustrate the relative values of pAKT(T308)/AKT and pAKT(S473)/AKT, respectively, which were similarly quantified for the bands illustrated in FIG. 6A. Compound 3 and Compound 4 suppressed the activation of AKT, which is essential for cell survival.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2022-25433, filed on February 22, 2022, the entire disclosure of which is incorporated by reference herein.

### Industrial Applicability

The disclosure is useful in a medicine, especially a medicine for cancer treatment.

## Claims

1. An anticancer agent containing an ATP8B2 inhibitor.

2. The anticancer agent according to claim 1, wherein the ATP8B2 inhibitor is 9-(1-octylcyclopropyl)nonan-1-ol, (S)-3-((9-(1-octylcyclopropyl)nonyl)oxy)propane-1,2-diol, or a pharmacologically acceptable salt thereof.

3. An anticancer agent screening method, comprising:
an exposure step of exposing a cancer cell to a test substance;
a quantification step of quantifying a plasmalogen present in an outer leaflet of a cell membrane of the cancer cell; and
a step of comparing an amount of the plasmalogen quantified in the quantification step with an amount of a control.
